# EUROPÄISCHE PATENTSCHRIFT

(11) **EP 0 994 096 B1**
(45) Veröffentlichungstag und Bekanntmachung des Hinweises auf die Patenterteilung: **09.04.2003**
(21) Anmeldenummer: 99117120.8
(22) Anmeldetag: 27.08.1999
(51) Int. Cl.: C07C 67/343, C07C 69/34, C07C 67/333

(54) **Verfahren zur Herstellung von 2-Methyl-1,3-dicarbonsäureestern**
Process for the preparation of esters of 2-methyl-1,3-dicarboxylic acid
Procédé de préparation d'esters d'acide 2-méthyl-1,3-dicarboxylique

(30) Priorität: 12.10.1998 DE 19846903
(43) Veröffentlichungstag der Anmeldung: 19.04.2000
(73) Patentinhaber: Degussa AG, 40474 Düsseldorf (DE)
(72) Erfinder: Bauer, Frank, Dr., 53127 Bonn (DE)

(56) Entgegenhaltungen:
- DE-A- 3 326 635

## Beschreibung

Die vorliegende Erfindung betrifft ein Verfahren zur Herstellung von 2-Methyl-1,3-dicarbonsäureestern der allgemeinen Formel 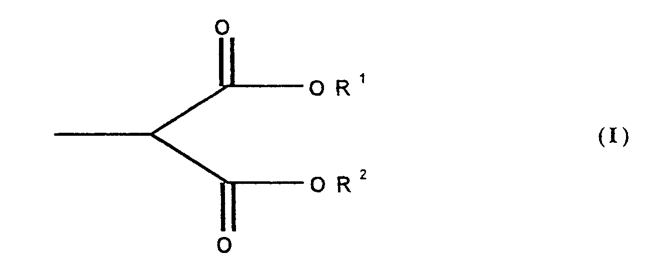 in der R¹ und R²
unabhängig voneinander für eine Alkyl-, Aralkyl-, Aryl- oder Cycloalkylgruppe mit 1 bis 12 Kohlenstoffatomen stehen bzw. gemeinsamer Teil einer Kohlenwasserstoffkette sind.

2-Methyl-1,3-dicarbonsäureester der allgemeinen Formel I und insbesondere der 2-Methyl-1,3-dicarbonsäurediethylester sind als organische Zwischenprodukte von großem Interesse.

Literaturbekannte Verfahren zur Herstellung der Verbindungen der allgemeinen Formel I gehen üblicherweise von den entsprechenden unsubstituierten 1,3-Dicarbonsäureestern der allgemeinen Formel 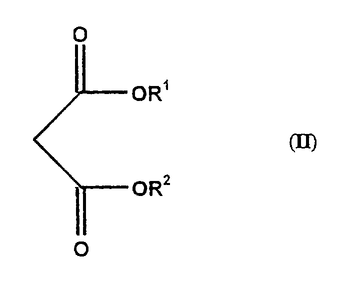 aus.

Bei deren Methylierung mit "klassischen" Alkylierungsmitteln wie Dimethylsulfat oder Methylbromid besteht die Schwierigkeit, daß in der Regel Gemische aus monomethylierten Produkten, dimethylierten Produkten und den unsubstituierten Ausgangsmaterialien anfallen, welche destillativ nur unter hohem Aufwand getrennt werden können.

Besser geeignet zur Gewinnung der reinen Monomethylverbindungen der allgemeinen Formel I ist daher eine reduktive Alkylierung der Verbindungen der allgemeinen Formel II durch Umsetzung mit Formaldehyd unter hydrierenden Bedingungen.

Wie bereits in der DE-A-33 26 635 beschrieben, müssen dabei ganz bestimmte Reaktionsbedingungen eingehalten werden, um relativ hohe Umsätze zu erzielen. Konkret wird eine Dosierung der zu methylierenden Verbindung der allgemeinen Formel II zur Mischung der übrigen Reaktionsteilnehmer bei erhöhter Temperatur und in Gegenwart sowohl eines Knoevenagel- als auch eines Hydrierkatalysators beansprucht.

Auch bei dieser Vorgehensweise wird jedoch unter Verwendung der üblicherweise eingesetzten Mengen an Formaldehyd und Lösungsmittel kein völlig quantitativer Umsatz der Ausgangsverbindungen der allgemeinen Formel II erzielt. Die so hergestellten Verbindungen der allgemeinen Formel I können daher - je nach Reaktionsbedingungen und den bei der destillativen Produktisolierung gewählten Trennbedingungen - zum Beispiel 0,5 bis 2,0 % nicht umgesetztes Ausgangsmaterial der allgemeinen Formel II enthalten. Dies ist jedoch gerade vor dem Hintergrund eines Einsatzes der Verbindungen der allgemeinen Formel I als Vorprodukte für Pharmawirkstoffe oder Pflanzenschutzmittel völlig inakzeptabel.

Es bestand somit Bedarf nach einem Verfahren zur Herstellung von Verbindungen der allgemeinen Formel I aus den entsprechenden unsubstituierten 1,3-Dicarbonsäureestern, welches die Zielprodukte in hohen Ausbeuten und mit minimalen Gehalten an unmethyliertem Ausgangsmaterial liefert.

Gegenstand der Erfindung ist daher ein Verfahren zur Herstellung von 2-Methyl-1,3-dicarbonsäureestern, in der die in den Estergruppen enthaltenen Reste unabhängig voneinander für eine Alkyl-, Aralkyl-, Aryl- oder Cycloalkylgruppe mit 1 bis 12 Kohlenstoffatomen stehen bzw. gemeinsamer Teil einer Kohlenwasserstoffkette sind, durch Umsetzung von 1,3-Dicarbonsäureestern mit Formaldehyd und Wasserstoff, dadurch gekennzeichnet, daß, bezogen auf 1,0 Mol des Dicarbonsäureesters 1,0 bis 2,0 Mol Formaldehyd eingesetzt werden und das Reaktionsgemisch oder daraus isolierte Verbindungen anschließend einer Thermolyse bei Temperaturen von 50 °C bis 300 °C unterworden werden.

Beim erfindungsgemäßen Verfahren zur reduktiven Alkylierung von Malonestern kommt eine Katalysatorkombination zum Einsatz, die aus einem üblichen Hydrier- und einem üblichen Knoevenagel-Katalysator besteht. Der zur Kondensationsreaktion von Malonester und Formaldehyd erforderliche übliche Knoevenagel-Katalysator kann dabei von saurem oder basischem Charakter sein. Typische derartige Katalysatoren sind beispielsweise Pyridin und/oder aliphatische Amine.

Als Hydrierkatalysatoren können übliche Katalysatoren wie z. B. Raney-Nickel oder Edelmetalle wie Palladium, Platin oder Rhodium in reiner Form oder kombinierter Zusammensetzung, vorzugsweise auf geeigneten Trägermaterialien wie z.B. Aktivkohle oder Aluminiumoxid verwendet werden. Bevorzugt wird Palladium auf Aktivkohle eingesetzt.

Mit Bezug auf die DE-A-33 26 635 wurde zunächst überraschend gefunden, daß gute Ausbeuten von zum Beispiel 70 % der Theorie an den Verbindungen der allgemeinen Formel I auch dann erzielt werden können, wenn der Formaldehyd zu den übrigen Komponenten der Reaktionsmischung dosiert wird. Voraussetzung ist auch in diesem Fall, daß die übrigen Komponenten der Reaktionsmischung bereits auf die erforderliche Reaktionstemperatur erwärmt wurden. Die für den Dosiervorgang erforderlichen Zeiten sind keineswegs länger als bei einer Dosierung der Verbindungen der allgemeinen Formel II zu den übrigen Komponenten der Reaktionsmischung und betragen bei Durchführung der Umsetzung im Labormaßstab üblicherweise weniger als 10 Stunden, bevorzugt zwischen 0,3 und 1,5 Stunden.

Erwartungsgemäß konnten, unabhängig davon, ob der Formaldehyd oder die Verbindung der allgemeinen Formel II zu den übrigen Komponenten der Reaktionsmischung dosiert wurde, durch Wahl eines ausreichenden Formaldehyd-Überschusses praktisch quantitative Umsätze der Verbindungen der allgemeinen Formel II von zum Beispiel > 99,95 % erzielt werden. Der Formaldehyd wird in Mengen von 1,0 bis 2,0 Mol, insbesondere 1,25 bis 1,50 Mol pro Mol 1,3-Dicarbonsäureester zudosiert. Die Ausbeuten an den Verbindungen der allgemeinen Formel I gingen jedoch mit zunehmendem Formaldehyd-Überschuß, bezogen auf die eingesetzte Menge an den Verbindungen der allgemeinen Formel II, drastisch zurück, während in zunehmendem Maße die Bildung von 2-Hydroxymethyl-2-methyl-1,3-dicarbonsäureestem der allgemeinen Formel III festgestellt wurde.

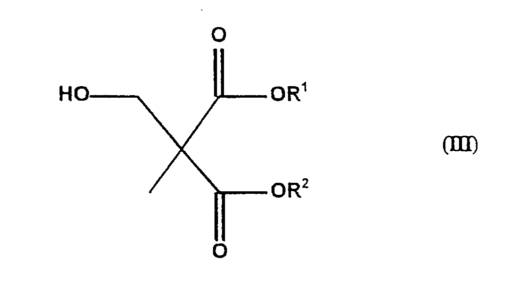

So handelt es sich auch bei dem in der DE-A-33 26 635 für die reduktive Alkylierung von Malonsäurediethylester mit Formaldehyd und Wasserstoff beschriebenen Nebenprodukt nicht um "Methoxymethylmalonsäurediethylester", sondern um 2-Hydroxymethyl-2-methylmalonsäurediethylester.

Die Bildung der Produkte der allgemeinen Formel III unter den Reaktionsbedingungen der reduktiven Alkylierung kann zwar durch eine starke Verdünnung des Systems mit geeigneten Lösungsmitteln wie Ethanol oder Essigsäure zurückgedrängt werden, auch verbessert sich der Umsatz bei gleichem Molverhältnis von Formaldehyd zu den Ausgangsverbindungen der allgemeinen Formel II. Die zum Erzielen von praktisch vollständigen Umsätzen erforderlichen Lösungsmittelmengen verschlechtern die Raum-Zeit-Ausbeute des Verfahrens aber derart, daß es für die Herstellung der Verbindungen der allgemeinen Formel I im technischen Maßstab ungeeignet ist.

Weitere Ausbeuteverluste können aus Esterkondensationen unter Beteiligung der Hydroxymethylgruppe der Verbindung der allgemeinen Formel III resultieren und werden insbesondere bei Temperaturen von > 100 °C beobachtet, welche im Sinne der Erzielung hoher Raum-Zeit-Ausbeuten anzustreben sind. Hierin ist auch eine Einschränkung bezüglich der möglichen Bedingungen für eine destillative Trennung der Verbindungen der allgemeinen Formel I von den als Nebenprodukt gebildeten Verbindungen der allgemeinen Formel III zu sehen. Eine weitere Einschränkung ergibt sich aus der Beobachtung, daß während der Destillation von Gemischen der Verbindungen der allgemeinen Formel I und Verbindungen der allgemeinen Formel III bei technisch bedeutenden Temperaturen von > 50 °C durch Zersetzungsreaktionen immer auch Formaldehyd freigesetzt wird, welcher neben einer inakzeptablen Verunreinigung des Produktes zu Belegungen und schlimmstenfalls Verstopfungen von Anlageteilen führen kann.

Überraschenderweise können die zum Erzielen eines praktisch quantitativen Umsatzes der Verbindungen der allgemeinen Formel II erforderlichen Mengen an Formaldehyd ohne Ausbeuteverluste eingesetzt werden, wenn das Reaktionsgemisch anschließend einer Thermolyse bei 50 °C bis 300 °C, vorzugsweise 100 °C bis 200 °C, besonders bevorzugt 130 °C bis 170 °C unterworfen wird.

Grundsätzlich ist es auch möglich, die unter den bevorzugten Reaktionsbedingungen der reduktiven Alkylierung in verstärktem Maße gebildeten Verbindungen der allgemeinen Formel III zu isolieren und gesondert einer Thermolyse zu unterwerfen. In beiden Fällen hat es sich als vorteilhaft erwiesen, unter Mitverwendung eines geeigneten Lösungsmittels, welches unter den Reaktionsbedingungen nicht nennenswert mit anderen Komponenten der Reaktionsmischung reagiert, zu arbeiten. Als solche kommen in Frage: gegebenenfalls halogenierte (aromatische oder aliphatische) Kohlenwasserstoffe, Alkanole (für R¹ ≡ R² idealerweise R¹OH, da dann keine Bildung gemischter Malonester möglich ist), Carbonsäuren, Ether, cyclische Ether und Polyether wie Diethylenglycoldiethylether. Insbesondere ist Ethanol oder Essigsäure geeignet. Auch Lösungsmittelgemische sind möglich. Die Lösungsmittel sollten jedoch eine ausreichende Wasserlöslichkeit besitzen, da bei der Reaktion gebildetes Wasser sonst unter Umständen zu Störungen am Hydrierkontakt führen kann.

Im Sinne einer Minimierung der Verfahrensschritte wird das Verfahren vorteilhaft derart durchgeführt, daß als Lösungsmittel für den Thermolyseschritt dasselbe Lösungsmittel beziehungsweise Lösungsmittelgemisch verwendet wird, wie es für die Umsetzung der Verbindungen der allgemeinen Formel II mit Formaldehyd und Wasserstoff zum Einsatz kommt. In besonders vorteilhafter Weise kann aber auch auf eine Separierung und gesonderte Thermolyse der Verbindungen der allgemeinen Formel III verzichtet werden. Hierzu wird die Reaktionsmischung, welche die Verbindungen der allgemeinen Formeln I und III, das Lösungsmittel, den Hydrierkatalysator und den Knoevenagel-Katalysator enthält, ohne weitere Aufarbeitung oder nach lediglich erfolgter Abtrennung des in der Regel festen Hydrierkatalysators der Thermolyse unterworfen.

Bei der genannten Vorgehensweise kann die Befreiung des Lösungsmittels von gelöstem Formaldehyd auf kostengünstige und ressourcenschonende Weise derart erfolgen, daß der gelöste Formaldehyd zur erneuten Umsetzung mit Verbindungen der allgemeinen Formel II genutzt wird.

Voraussetzung für eine erfolgreiche Rückführung des Lösungsmittels oder Lösungsmittelgemisches ist in jedem Fall, daß das bei der reduktiven Alkylierung gebildete Wasser entweder vor der Thermolyse zumindest teilweise abgetrennt wird oder daß eine zumindest partielle Entwässerung des Lösungsmittels beziehungsweise Lösungsmittelgemisches vor dessen Wiederverwendung erfolgt.

Bevorzugte Lösungsmittel beziehungsweise Lösungsmittelgemische für den Thermolyseschritt sind daher solche, welche auf einfache Weise von Wasser befreit werden können.

Die Selektivität der Thermolysereaktion, das heißt die Bildung von 2-Methyl-1,3-dicarbonsäureestern auf Kosten von Oligomeren und Polymeren, sowie die Reaktionsgeschwindigkeit lassen sich durch den Zusatz geeigneter Katalysatoren verbessern. Als besonders geeignet haben sich Kaliumacetat und insbesondere kupferhaltige Katalysatoren wie Kupfersalze, insbesondere Kupfer-II-acetat, welche in der Reaktionsmischung homogen gelöst sind, herausgestellt. Es können aber auch einfacher zu handhabende und abzutrennende Festbettkatalysatoren wie Aluminiumoxide oder auf einem Trägermaterial fixierte Katalysatoren, insbesondere Kupfer-haltige, eingesetzt werden.

Üblicherweise genügen Katalysatormengen von 0,01 bis 50,0 g, bevorzugt 0,05 g bis 5,0 g pro Mol eingesetzter Verbindung der allgemeinen Formel II. Besonders bevorzugt werden 0,1 bis 2,0 g, ganz besonders bevorzugt 0,5 g bis 1,6 g Katalysator pro Mol der Verbindung der allgemeinen Formel II verwendet.

Beim Einsatz von Kupfer-II-acetat als Thermolysekatalysator hat sich eine portionsweise oder - bevorzugt - kontinuierliche Zugabe des Katalysators als vorteilhaft herausgestellt. Lagert man nämlich die mit dem Katalysator sowie - zur Erhöhung der Löslichkeit des Katalysators - optional mit einer Säure wie Essigsäure versetzte Reaktionsmischung der reduktiven Alkylierung über einen Zeitraum von mehreren Stunden, so verringerten sich die Thermolyseausbeuten deutlich.

Da die Thermolysereaktion in Gegenwart von Katalysatoren und insbesondere bei Temperaturen von > 80 °C recht schnell verläuft, wird das erfindungsgemäße Verfahren vorteilhafterweise kontinuierlich durchgeführt. Hierzu kann anstatt eines einzelnen Reaktors zum Beispiel eine zweistufige Rührkesselkaskade eingesetzt werden. Als noch günstiger hat sich in der Praxis aber der Einsatz eines Fallfilm-, Dünnschicht- oder Kurzwegverdampfers als Thermolysereaktor bewährt.

Neben dem Vorteil eines kontinuierlichen und damit besonders wirtschaftlichen Betriebes können so durch den Fachmann unschwer Bedingungen eingestellt werden, welche eine schnelle Entfernung des 2-Methyl-1,3-dicarbonsäureesters aus der Reaktionszone und damit eine geringe Nebenproduktbildung bewirken sowie gleichzeitig die Rückreaktion der Verbindungen der allgemeinen Formel I mit freigesetztem Formaldehyd unterdrücken.

Um eine ausreichend schnelle Verdampfung der Verbindungen der allgemeinen Formel I zu erzielen, muß hierzu in der Regel am Verdampfer ein Vakuum angelegt werden. Bevorzugt wird die Thermolyse daher unter einem verminderten Druck von 5 mbar bis 900 mbar, besonders bevorzugt von 100 mbar bis 300 mbar durchgeführt.

So liegen im Falle der Thermolyse eines Gemisches aus 2-Hydroxymethyl-2-methyldicarbonsäurediethylester und 2-Methyl-1,3-dicarbonsäurediethylester die Ausbeuten an reinem 2-Methyl-1,3-dicarbonsäurediethylester (Gehalt an unsubstituiertem 1,3-Dicarbonsäurediethylester < 500 ppm) um bis zu 6 % über denjenigen Ausbeuten, welche erhalten werden, wenn die Thermolyse unter Rühren in Lösung durchgeführt wird.
Ähnlich gute Ausbeuten und Produktreinheiten lassen sich erzielen, wenn die Verbindungen der allgemeinen Formel III beziehungsweise Gemische, welche die Verbindungen der allgemeinen Formel III enthalten, unter Abdestillieren der Verbindungen der allgemeinen Formel I kontinuierlich in einen heißen Thermolysesumpf dosiert werden.

In der Praxis hat sich gezeigt, daß insbesondere bei der kontinuierlichen Thermolyse der Verbindungen der allgemeinen Formel III nur dann sehr hohe und wirtschaftlich attraktive Durchsätze erzielt werden, wenn ein geringer Gehalt des Thermolysates an den Verbindungen der allgemeinen Formel III zugelassen wird.

Wie oben bereits erläutert, kann es wegen der thermischen Empfindlichkeit der Verbindungen der allgemeinen Formel III dann aber zu Schwierigkeiten bei der destillativen Isolierung der Produkte der allgemeinen Formel I kommen.

Erfreulicherweise hat sich jedoch gezeigt, daß auch bei hohen Durchsätzen Thermolysate mit geringen Gehalten an den Verbindungen der allgemeinen Formel III erhalten werden können, wenn die Thermolyse derart durchgeführt wird, daß die Brüden, welche die Thermolysezone verlassen, einer Fraktionierung unterworfen werden und die an den Verbindungen der allgemeinen Formel III reiche Fraktion - gegebenenfalls nach Ergänzung von Katalysator und/oder Lösungsmittel - in die Thermolysezone zuruckgeführt wird. Im einfachsten Fall erfolgt die Thermolyse daher unter Einsatz eines Dünnschichtverdampfers als Reaktor, wobei in der Brüdenleitung eine Rektifizier-Einheit, bestehend zum Beispiel aus Glockenböden oder Füllkörpern, vorhanden ist. Der Rücklauf der Rektifizier-Einheit gelangt so erneut in die heiße Thermolysezone.

Alternativ kann der Rücklauf der Rektifizier-Einheit in die Vorlage des Thermolysereaktors gepumpt werden, wo auf besonders einfache Weise eine erneute Vermischung mit dem Katalysator stattfindet.

Im Thermolysat verbliebene Anteile an den Verbindungen der allgemeinen Formel III können schließlich durch Derivatisierung mit geeigneten Derivatisierungsmitteln, welche die Verbindung III in ein temperaturstabiles und damit destillierbares Folgeprodukt überführen, wie z. B. organische Säuren wie Essigsäure und deren Anhydride, höhere (Carbon)Säuren, Säurechloride und Silylierungsmittel, wie insbesondere Trimethylsilylchlorid, in thermisch stabile Folgeprodukte überführt werden, so daß auch unter solchen Bedingungen, welche im großtechnischen Maßstab anzustreben sind, eine problemlose Isolierung der Verbindungen der allgemeinen Formel I durch fraktionierte Destillation möglich ist. Bevorzugtes Derivatisiserungsmittel ist Essigsäureanhydrid.

Die folgenden Beispiele sollen das Verfahren nach der Erfindung weiter erläutern, nicht aber dessen Anwendungsbereich limitieren.

### Beispiel 1: (Vergleichsbeispiel) 2-Methyl-1,3-dicarbonsäurediethylester

In einem 1,5-l-Hubrühr-Autoklaven wurde unter Rühren eine Mischung aus 320,0 g 1,3-Dicarbonsäurediethylester, 78,5 g Ethanol, 8,6 g Pyridin und 8,0 g Pd/C (5 %) vorgelegt. Die im Autoklaven befindliche Luft verdrängte man durch dreimaliges Spülen mit Stickstoff und anschließendes Aufdrücken von bar Wasserstoff.

Dann wurde die Mischung auf 120 °C erhitzt und der Wasserstoffdruck auf 35 bar bis 40 bar adjustiert, bevor man innerhalb von 60 Minuten 243,0 g einer ethanolischen Formaldehydlösung (66,0 g p-HCHO in 177,0 g Ethanol; 10 % Überschuß) über eine Kolbenpumpe in den Autoklaven dosierte. Um die Gesamtmenge an Formaldehyd in den Autoklaven zu überführen, wurde das Dosiersystem anschließend mit 39,3 g Ethanol gespült.

Man ließ noch 80 Minuten bei 120 °C/35 bar nachreagieren, dann wurde der Ansatz auf Raumtemperatur abgekühlt und durch Filtration vom Katalysator befreit. Am Katalysator anhaftendes Produkt überführte man durch Waschen mit 39,3 g Ethanol in das Filtrat.

Dieses wurde bei 60 °C/ 10 mbar von Leichtsiedern befreit, wobei 338,0 g eines klaren, farblosen Rückstandes verblieben. Das gaschromatographisch ermittelte Produktverhältnis von 2-Methyl-1,3-dibarbonsäurediethylester zu 2-Hydroxymethyl-2-methyl-1,3-dicarbonsäurediethylester betrug auf der Basis der FID-Flächenprozente nach Silylierung etwa 7 : 1. Der Gehalt des Rückstandes an nicht umgesetztem 1,3-Dicarbonsäurediethylester wurde nach Silylierung zu 3,0 FID-Flächenprozent ermittelt.

### Beispiel 2: 2-Methyl-1,3-dicarbonsäurediethylester

### Hydrierschritt:

In einem 5-l-Hubrühr-Autoklaven wurde unter Rühren eine Mischung aus 1600,0 g 1,3-Dicarbonsäurediethylester, 392,5 g Ethanol, 86,0 g Triethylamin und 40,0 g Pd/C (5 %) vorgelegt. Die im Autoklaven befindliche Luft verdrängte man durch dreimaliges Spülen mit Stickstoff und anschließendes Aufdrücken von 5 bar Wasserstoff.

Dann wurde die Mischung auf 120 °C erhitzt und der Wasserstoffdruck auf 35 bar bis 40 bar adjustiert, bevor man innerhalb von 60 Minuten 1403,0 g einer ethanolischen Formaldehydlösung (400,8 g p-HCHO in 1002,2 g Ethanol; 33 % Überschuß) über eine Kolbenpumpe in den Autoklaven dosierte. Um die Gesamtmenge an Formaldehyd in den Autoklaven zu überführen, wurde das Dosiersystem anschließend mit 39,3 g Ethanol gespült.

Man ließ noch 80 Minuten bei 120 °C/35 bar nachreagieren, dann wurde der Ansatz auf Raumtemperatur abgekühlt und durch Filtration vom Katalysator befreit. Am Katalysator anhaftendes Produkt überführte man durch Waschen mit 39,3 g Ethanol in das Filtrat. Auf diese Weise wurden 3559,6 g einer klaren, annähernd farblosen Lösung von 2-Methyl-1,3-dicarbonsäurediethylester und 2-Hydroxymethyl-2-methyl-1,3-dicarbonsäurediethylester in Ethanol/Triethylamin erhalten. Das gaschromatographisch ermittelte Produktverhältnis von 2-Methyl-1,3-dicarbonsäurediethylester zu 2-Hydroxymethyl-2-methyl-1,3-dicarbonsäurediethylester betrug auf der Basis der FID-Flächenprozente nach Silylierung etwa 3 : 1.

### Thermolyseschritt:

1423,8 g des filtrierten Hydrieraustrages wurden unter Rühren mit 100,0 g Essigsäure (96 %ig) sowie 8,0 g Kupfer-II-acetat-Hydrat versetzt und anschließend 15 Minuten bei 45 °C kräftig gerührt, um den Katalysator vollständig in Lösung zu bringen.

Die erhaltene türkis-farbene Lösung dosierte man bei einem Vakuum von etwa 90 mbar in einen 250-ml-Thermolysekolben mit aufgesetztem Spritzschutz und 30-cm-Füllkörperkolonne. Der Thermolysekolben, in dem sich zur Durchmischung des während der Thermolyse anfallenden - in der Wärme niedrigviskosen - Sumpfes ein Magnetrührkern befand, wurde mittels eines Ölbades von 200 °C beheizt.

Die Dosierungsgeschwindigkeit wurde derart geregelt, daß die Innentemperatur (Gasphase und später auch Sumpf) etwa 130 °C betrug. Unter den genannten Bedingungen nahm die Thermolyse etwa 5 Stunden in Anspruch.

Die am Kopf der Füllkörperkolonne anfallenden Brüden wurden über ein unbeheiztes Brüdenrohr in eine weitere Füllkörperkolonne mit einem Abtriebsteil von 30 cm und einem Verstärkerteil von 60 cm geleitet.

In dieser Kolonne erfolgte die Trennung der Leichtsieder (Ethanol, Wasser, HCHO) vom 2-Methyl-1,3-dicarbonsäurediethylester, welcher aus dem Sumpf der Kolonne abgezogen wurde.

Auf diese Weise konnten 78 % der Theorie an 2-Methyl-1,3-dicarbonsäurediethylester, bezogen auf die eingesetzte Menge an 1,3-Dicarbonsäurediethylester, mit einem Gehalt an unsubstituiertem 1,3-Dicarbonsäurediethylester von < 500 ppm erhalten werden.

### Beispiel 3: 2-Methyl-1,3-dicarbonsäurediethylester

### Hydrierschritt:

Es wurde wie in Beispiel 2 verfahren.

### Thermolyseschritt:

Jeweils 500,0 g beziehungsweise 423,8 g des filtrierten Hydrieraustrages wurden unter Rühren mit insgesamt 40,0 g Essigsäure (96 %ig) sowie insgesamt 6,5 g Kupfer-II-acetat-Hydrat versetzt und anschließend jeweils 5 Minuten bei 45 °C kräftig gerührt, um den Katalysator vollständig in Lösung zu bringen.

Die erhaltene türkis-farbene Lösung dosierte man bei einer Manteltemperatur des Verdampfers von 220 °C und einem Vakuum von etwa 155 mbar mit einer Dosierrate von etwa 1100 g/Stunde kontinuierlich auf einen Dünnschichtverdampfer (Verdampferfläche: 700 cm²) mit aufgesetzter 30-cm-Füllkörperkolonne. Nach kontinuierlicher Abtrennung der Leichtsieder durch Einspeisen der Brüden in eine Kolonne mit Abtriebs- und Verstärkerteil wurden 80 % der Theorie an 2-Methyl-1,3-dicarbonsäurediethylester, bezogen auf die eingesetzte Menge an 1,3-Dicarbonsäurediethylester, mit einem Gehalt an unsubstituiertem 1,3-Dicarbonsäurediethylester von < 500 ppm erhalten.

### Beispiel 4: 2-Methyl-1,3-dicarbonsäurediethylester

### Hydrierschritt:

Es wurde wie in Beispiel 2 verfahren.

### Thermolyseschritt:

Es wurde analog zu Beispiel 2 verfahren, jedoch erfolgte eine Rückführung des Rücklaufes der für die Trennung von 2-Hydroxymethyl-2-methyl-1,3-dicarbonsäurediethylester und 2-Methyl-1,3-dicarbonsäurediethylester eingesetzten Kolonne in die Dosiervorlage des Verdampfers. Auf diese Weise konnte eine Ausbeute von 85 % der Theorie an 2-Methyl-1,3-dicarbonsäurediethylester, bezogen auf die eingesetzte Menge an 1,3-Dicarbonsäurediethylester, mit einem Gehalt an unsubstituiertem 1,3-Dicarbonsäurediethylester von < 500 ppm erhalten Werden.

### Beispiel 5: 2-Methyl-1,3-dicarbonsäurediethylester

### Hydrierschritt:

Es wurde wie in Beispiel 2 verfahren.

### Thermolyseschritt:

1423,8 g des filtrierten Hydrieraustrages wurden destillativ vom Lösungsmittel befreit und unter Rühren mit 40,0 g Essigsäure (96 %ig) sowie 8,0 g Kupfer-II-acetat-Hydrat versetzt. Die so erhaltene Lösung wurde zwei Stunden bei 120 °C gerührt und dann destillativ vom Katalysator abgetrennt.

Auf diese Weise konnte eine Ausbeute an 2-Methyl-1,3-dicarbonsäurediethylester von 76 % der Theorie, bezogen auf die eingesetzte Menge an 1,3-Dicarbonsäurediethylester, mit einem Gehalt an unsubstituiertem 1,3-Dicarbonsäurediethylester von < 500 ppm erhalten werden.

## Patentansprüche

1. Verfahren zur Herstellung von 2-Methyl-1,3-dicarbonsäureestern der allgemeinen Formel I 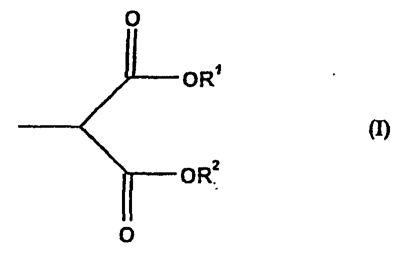 in der R¹ und R² unabhängig voneinander für eine Alkyl-, Aralkyl-, Aryl- oder Cycloalkylgruppe mit 1 bis 12 Kohlenstoffatomen stehen bzw. gemeinsamer Teil einer Kohlenwasserstoffkette sind, durch Umsetzung von 1,3-Dicarbonsäureestern der allgemeinen Formel II 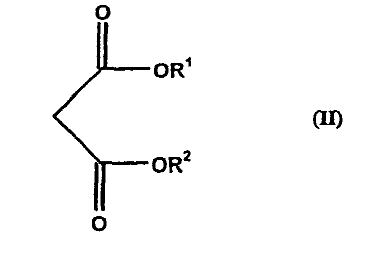 mit Formaldehyd und Wasserstoff in Gegenwart eines Knoevenagel-Katalysators von saurem oder basischem Charakter und eines Hydrierkatalysators,
**dadurch gekennzeichnet,**
**daß**, bezogen auf 1,0 Mol des Dicarbonsäureesters der allgemeinen Formel II 1,0 bis 2,0 Mol Formaldehyd eingesetzt werden und das Reaktionsgemisch oder daraus isolierte Verbindungen der allgemeinen Formel III 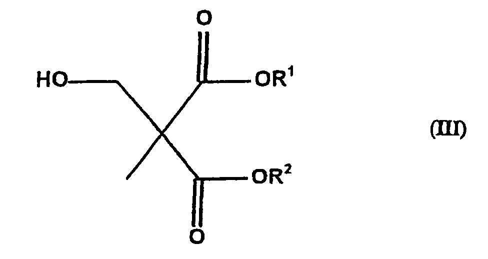 anschließend einer Thermolyse bei Temperaturen von 50 °C bis 300 °C unterworfen werden.

2. Verfahren nach Anspruch 1,
**dadurch gekennzeichnet,**
**daß** die Thermolyse des Reaktionsgemisches oder der isolierten Verbindung der allgemeinen Formel III in Gegenwart eines Lösungsmittels oder Lösungsmittelgemisches durchgeführt wird, das vorzugsweise identisch mit jenem ist, welches auch bei der vorausgegangenen Reaktion verwendet wurde.

3. Verfahren nach Anspruch 1,
**dadurch gekennzeichnet,**
**daß** die Thermolyse in Gegenwart eines homogen gelösten Katalysators durchgeführt wird, wobei der Katalysator in Mengen von 0,01 g bis 50,0 g, vorzugsweise 0,1 bis 2 g und insbesondere 0,5 bis 1,6 g pro Mol des zur Methylierung eingesetzten 1,3-Dicarbonsäureesters der allgemeinen Formel II verwendet wird.

4. Verfahren nach Anspruch 3,
**dadurch gekennzeichnet,**
**daß** der homogen gelöste Katalysator eine Kupferverbindung ist.

5. Verfahren nach Anspruch 1 oder 2,
**dadurch gekennzeichnet,**
**daß** die Thermolyse in Gegenwart eines auf einem Trägermaterial fixierten, vorzugsweise Kupfer-haltigen Katalysator durchgeführt wird.

6. Verfahren nach einem der vorhergehenden Ansprüche,
**dadurch gekennzeichnet,**
**daß** die Thermolyse unter einem verminderten Druck von 5 mbar bis 900 mbar durchgeführt wird.

7. Verfahren nach einem der vorhergehenden Ansprüche,
**dadurch gekennzeichnet,**
**daß** die zur Thermolyse eingesetzte Lösung in ein über einen Festbettkatalysator im Kreis geführtes, auf Thermolysetemperatur erhitztes, unter den Thermolysebedingungen hinreichend stabiles Reaktionsmedium eingespeist wird.

8. Verfahren nach einem der Ansprüche 1 bis 6,
**dadurch gekennzeichnet,**
**daß** die zur Thermolyse eingesetzte Lösung in ein den homogen gelösten Katalysator enthaltendes, auf Thermolysetemperatur erhitztes, unter den Thermolysebedingungen hinreichend stabiles Reaktionsmedium eingespeist wird, das über einen großflächigen Wärmetauscher, vorzugsweise vom Typ eines Fallfilm-, Dünnschicht- oder Kurzwegverdampfers im Kreis geführt wird.

9. Verfahren nach einem der vorhergehenden Ansprüche,
**dadurch gekennzeichnet,**
**daß** die Thermolyse kontinuierlich durchgeführt wird..

10. Verfahren nach einem der vorhergehenden Ansprüche,
**dadurch gekennzeichnet,**
**daß** auf eine Isolierung der Verbindungen der allgemeinen Formel III verzichtet und das Reaktionsgemisch der Umsetzung eines 1,3-Dicarbonsäureesters der allgemeinen Formel II mit Formaldehyd und Wasserstoff, ohne oder mit Abtrennung des Hydrierkatalysators, direkt der Thermolyse unterworfen wird.

11. Verfahren nach einem der vorhergehenden Ansprüche,
**dadurch gekennzeichnet,**
**daß** bei der Thermolyse nicht umgesetzte Verbindungen der allgemeinen Formel III ohne oder mit Katalysatorzugabe, erneut einer Thermolyse unterworden werden.

12. Verfahren nach einem der vorhergehenden Ansprüche,
**dadurch gekennzeichnet,**
**daß** bei der Thermolyse nicht umgesetzte Verbindungen der allgemeinen Formel III durch Umsetzung mit organischen Säuren oder deren Anhydriden, Säurechloriden oder Silylierungsmitteln in temperaturstabile, destillativ abtrennbare Folgeprodukte überführt werden.

13. Verfahren nach einem der vorhergehenden Ansprüche,
**dadurch gekennzeichnet,**
**daß** bei der Thermolyse nicht umgesetzte Verbindungen der allgemeinen Formel III durch Umsetzung mit Essigsäureanhydrid in destillativ abtrennbare Folgeprodukte überführt werden.

## Claims

1. A process for the preparation of a 2-methyl-1,3-dicarboxylate of the general formula I 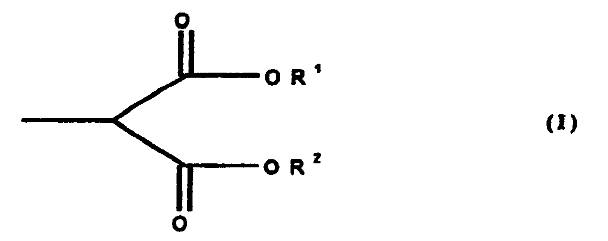 in which R¹ and R² independently of one another are an alkyl, aralkyl, aryl or cycloalkyl group having from 1 to 12 carbon atoms or are a common part of a hydrocarbon chain, by reaction of a 1,3-dicarboxylate of the general formula II 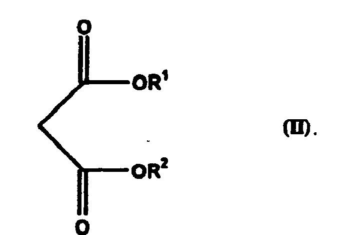 with formaldehyde and hydrogen in the presence of a Knoevenagel catalyst which is acidic or basic in character and a hydrogenation catalyst,
**characterized in that**, based on 1.0 mol of the dicarboxylate of the general formula II, from 1.0 to 2.0 mol of formaldehyde are used, and the reaction mixture or compound of the general formula III 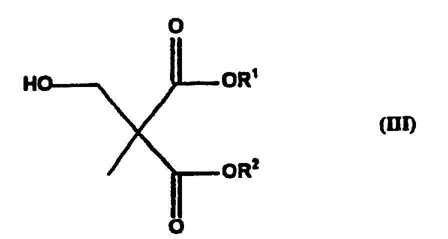 isolated therefrom is then subjected to thermolysis at temperatures of from 50°C to 300°C.

2. A process according to claim 1,
**characterized in that**
the thermolysis of the reaction mixture or of the isolated compound of the general formula III is carried out in the presence of a solvent or solvent mixture which is preferably identical to that already used in the preceding reaction.

3. A process according to claim 1,
**characterized in that**
the thermolysis is carried out in the presence of a homogeneously dissolved catalyst, the catalyst being used in amounts of from 0.01 g to 50.0 g, preferably from 0.1 to 2 g and in particular from 0.5 to 1.6 g, per mole of the 1,3-dicarboxylate of the general formula II used for the methylation.

4. A process according to claim 3,
**characterized in that**
the homogeneously dissolved catalyst is a copper compound.

5. A process according to claim 1 or 2,
**characterized in that**
the thermolysis is carried out in the presence of a, preferably copper-containing, catalyst fixed to a support material.

6. A process according to any one of the preceding claims,
**characterized in that**
the thermolysis is carried out under a reduced pressure of from 5 mbar to 900 mbar.

7. A process according to any one of the preceding claims,
**characterized in that**
the solution used for the thermolysis is fed into a reaction medium which is circulated over a fixed-bed catalyst and heated to thermolysis temperature, and is sufficiently stable under the thermolysis conditions.

8. A process according to any one of claims 1 to 6,
**characterized in that**
the solution used for the thermolysis is fed into a reaction medium which contains the homogeneously dissolved catalyst, is heated to thermolysis temperature and is sufficiently stable under the thermolysis conditions, and which is circulated over a large-area heat exchanger, preferably of the falling-film, thin-layer or short-path evaporator type.

9. A process according to any one of the preceding claims,
**characterized in that**
the thermolysis is carried out continuously.

10. A process according to any one of the preceding claims,
**characterized in that**
isolation of the compound of the general formula III is dispensed with, and the reaction mixture of the reaction of a 1,3-dicarboxylate of the general formula II with formaldehyde and hydrogen is subjected directly, with or without removal of the hydrogenation catalyst, to thermolysis.

11. A process according to any one of the preceding claims,
**characterized in that**
a compound of the general formula III unreacted during the thermolysis is again subjected, with or without the addition of catalysts, to thermolysis.

12. A process according to any one of the preceding claims,
**characterized in that**
a compound of the general formula III unreacted during the thermolysis is converted, by reaction with organic acids or anhydrides thereof, acid chlorides or silylating agents, into a temperature-stable secondary product which can be removed by distillation.

13. A process according to any one of the preceding claims,
**characterized in that**
a compound of the general formula III unreacted during the thermolysis is converted, by reaction with acetic anhydride, into a secondary product which can be removed by distillation.

## Revendications

1. Procédé de préparation d'esters d'acide 2-méthyl-1,3-dicarboxylique de formule générale I, 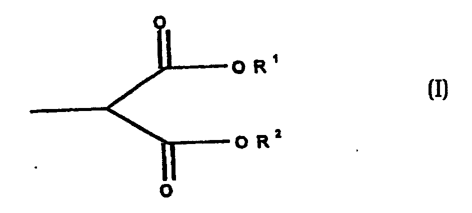 dans laquelle R¹ et R² indépendamment l'un de l'autre, représentent un groupe allyle, aralkyle, aryle ou cycloalkyle ayant de 1 à 12 atomes de carbone, ou sont une partie conjointe d'une chaîne hydrocarbonée par réaction d'esters d'acide 1,3-dicarboxylique de formule générale II 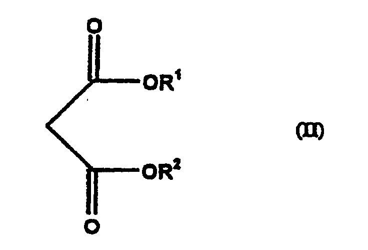 avec le formaldéhyde et l'hydrogène en présence d'un catalyseur de Knoevenagel de caractère acide ou basique et d'un catalyseur d'hydrogénation,
**caractérisé en ce que**
rapporté à 1,0 mol d'ester d'acide dicarboxylique de formule générale II on met en oeuvre de 1,0 à 2,0 mol de formaldéhyde et on soumet le mélange réactionnel ou les composés qui en sont isolés de formule générale III, 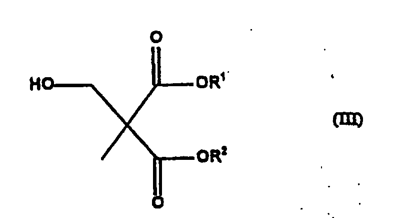 ensuite à une thermolyse à des température allant de 50 à 300°C.

2. Procédé selon la revendication 1,
**caractérisé en ce que**
la thermolyse du mélange réactionnel ou du composé isolé de formule générale III est effectuée en présence d'un solvant ou d'un mélange de solvants qui est de préférence identique à celui qui a été utilisé également au cours de la réaction précédente.

3. Procédé selon la revendication 1,
**caractérisé en ce que**
la thermolyse s'effectue en présence d'un catalyseur homogène dissout, dans laquelle le catalyseur est utilisé en quantités allant de 0,01 g à 50,0 g de préférence de 0,1 à 2 g et en particulier de 0,5 à 1,6 g par mol d'ester d'acide 1,3-dicarboxylique mis en oeuvre en vue de la méthylation de formule générale III.

4. Procédé selon la revendication 3,
**caractérisé en ce que**
le catalyseur homogène dissout est un composé du cuivre.

5. Procédé selon l'une quelconque des revendications 1 ou 2,
**caractérisé en ce que**
la thermolyse est effectuée en présence d'un catalyseur contenant du cuivre, de préférence fixé sur un matériau de support.

6. Procédé selon l'une quelconque des revendications précédentes,
**caractérisé en ce qu'**
on effectue la thermolyse sous une pression réduite de 5 mbars à 900 mbars.

7. Procédé selon l'une quelconque des revendications précédentes,
**caractérisé en ce que**
la solution mise en oeuvre en vue de la thermolyse, est chargée dans un milieu réactionnel suffisamment stable dans les conditions de la thermolyse, chauffé à la température de thermolyse et recyclé sur un catalyseur à lit fixe.

8. Procédé selon l'une quelconque des revendications 1 à 6,
**caractérisé en ce que**
la solution mise en oeuvre en vue de la thermolyse est chargée dans un milieu réactionnel suffisamment stable dans les conditions de thermolyse, chauffé à la température de thermolyse, et contenant le catalyseur dissout homogène, milieu qui est recyclé par l'intermédiaire d'un échangeur de chaleur à grande surface, de préférence du type d'un évaporateur en film tombant, en couche mince, ou par distillation moléculaire.

9. Procédé selon l'une quelconque des revendications précédentes,
**caractérisé en ce qu'**
on effectue la thermolyse en continu.

10. Procédé selon l'une quelconque des revendications précédentes,
**caractérisé en ce qu'**
on renonce à un isolement des composés de formule générale III,
et on soumet le mélange réactionnel de la condensation d'un ester d'acide 1,3-dicarboxylique de formule générale III, avec le formaldéhyde et l'hydrogène, sans ou avec séparation du catalyseur d'hydrogénation, directement à la thermolyse.

11. Procédé selon l'une quelconque des revendications précédentes,
**caractérisé en ce que**
les composés qui n'ont pas réagi au cours de la thermolyse, de formule générale III sont soumis à nouveau à une thermolyse.

12. Procédé selon l'une quelconque des revendications précédentes,
**caractérisé en ce qu'**
on convertit les composés qui n'ont pas réagi au cours de la thermolyse de formule générale III en sous-produits stables à la chaleur, séparables par distillation, par réaction avec des acides organiques ou leur anhydride, leur chlorure d'acide ou avec des agents de silylation.

13. Procédé selon l'une quelconque des revendications précédentes,
**caractérisé en ce qu'**
on convertit les composés qui n'ont pas réagi au cours de la thermolyse, de formule générale III par réaction avec de l'anhydride acétique, en sous-produits séparables par distillation.
